# EUROPÄISCHE PATENTANMELDUNG

(11) **EP 0 529 462 A1**
(43) Veröffentlichungstag der Anmeldung: **03.03.1993**
(21) Anmeldenummer: 92114007.5
(22) Anmeldetag: 17.08.1992
(51) Int. Cl.: C07D 405/12, C07D 453/02, C07D 401/14, A61K 31/40, A61K 31/415

(54) **Benzodioxanderivate**

(30) Priorität: 22.08.1991 DE 4127849
(71) Anmelder: MERCK PATENT GmbH, D-64271 Darmstadt (DE)
(72) Erfinder: Böttcher, Henning, Dr., W-6100 Darmstadt (DE); Seyfried, Christoph, Dr., W-6104 Jugenheim (DE); Greiner, Hartmut, Dr., W-6100 Darmstadt (DE); Bartoszyk, Gerd, W-6100 Darmstadt (DE)

(57) **Zusammenfassung**

1,4-Benzodioxanderivate der Formel I 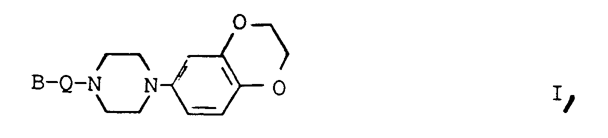
worin B und Q die in Patentanspruch 1 angegebenen Bedeutungen haben sowie deren Salze, zeigen Wirkungen auf das Zentralnervensystem.

## Beschreibung

Die Erfindung betrifft neue 1,4-Benzodioxanderivate der Formel I 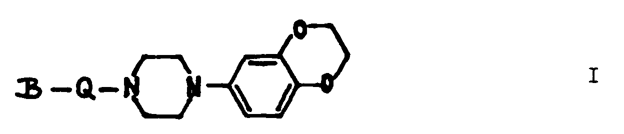
worin
- B: einen unsubstituierten oder einfach durch CN, CO-R¹, CₙH₂ₙ-R¹, Hal, OH, OA, O-CₙH₂ₙ-COR¹ oder NHR² substituierten Indol-3-yl- oder Benzimidazol-1-yl-rest,
- R¹: OH, OA, NH₂, NHA, NA₂, NH-3-chinuclidinyl,NH-CH₂-3-pyridinyl, NH-1-piperazinyl oder NH-4-(N',N''-di-carbethoxy-pyrazolidinyl),
- R²: H, A, CO-A, CO-Ar, CO-NH₂, CO-NHA, CO-NA₂, SO₂-Ar oder SO₂-A,
- Q: CₙH₂ₙ,
- n: 1, 2, 3, 4, 5 oder 6,
- A: Alkyl mit 1-6 C-Atomen,
- Ar: einen unsubstituierten oder einen ein- oder zweifach durch A, Hal, CN, OH und/oder OA substituierten Phenylrest,
und
- Hal: F, Cl, Br oder I
bedeuten
sowie deren Salze.

Der Erfindung lag die Aufgabe zugrunde, neue Verbindungen aufzufinden, die zur Herstellung von Arzneimitteln verwendet werden können.

Es wurde gefunden, daß die Verbindungen der Formel I und ihre physiologisch unbedenklichen Säureadditionssalze wertvolle pharmakologische Eigenschaften besitzen. So zeigen sie insbesondere Wirkungen auf das Zentralnervensystem, vor allem serotonin-agonistische und -antagonistische Wirkungen. Sie hemmen die Bindung von tritiierten Serotoninliganden an hippocampale Rezeptoren (Cossery et al., European J. Pharmacol. 140 (1987), 143-155). Außerdem treten Veränderungen der DOPA-Akkumulation im Striatum und der 5-HTP-Akkumulation in N.raphe auf (Seyfried et al., European J. Pharmacol. 160 (1989), 31-41). Weiterhin treten analgetische und blutdrucksenkende Wirkungen auf; so wird bei kathetertragenden wachen, spontan hypertonen Ratten (Stamm SHR/Okamoto/NIH-MO-CHB-Kisslegg; Methode vgl. Weeks und Jones, Proc. Soc. Exptl. Biol. Med. 104 (1960), 646-648) der direkt gemessene Blutdruck nach peroraler Gabe der Verbindungen gesenkt. Ebenso eignen sie sich als Prophylaxe und zur Bekämpfung der Folgen cerebraler Infarktgeschehen (Apoplexia cerebri), wie Schlaganfall und cerebraler Ischämien.

Verbindungen der Formel I und ihre physiologisch unbedenklichen Säureadditionssalze können daher als Arzneimittelwirkstoffe für Anxiolytika, Antidepressiva, Neuroleptika und/oder Antihypertonika, ferner zur Cerebroprotektion nach Schlaganfall bzw. zur Prophylaxe, bei Morbus Alzheimer und auch als Zwischenprodukte zur Herstellung anderer Arzneimittelwirkstoffe verwendet werden.

Gegenstand der Erfindung sind die 1,4-Benzodioxanderivate der Formel I sowie ihre physiologisch unbedenklichen Säureadditionssalze.

Der Rest A bedeutet Alkyl mit 1, 2, 3, 4, 5 oder 6, insbesondere 1 oder 2 C-Atomen, vorzugsweise Methyl, ferner auch Ethyl, n-Propyl, Isopropyl, n-Butyl, Isobutyl, sek.-Butyl oder tert.-Butyl. OA ist vorzugsweise Methoxy, ferner auch Ethoxy, n-Propoxy, Isopropoxy, n-Butoxy, Isobutoxy, sek.-Butoxy oder tert.-Butoxy. NHA ist vorzugsweise Methylamino, ferner Ethylamino, n-Propylamino, Isopropylamino, n-Butylamino, Isobutylamino, sek.-Butylamino oder tert.-Butylamino. NA₂ bedeutet vorzugsweise Dimethylamino, ferner N-Ethyl-N-methylamino, Diethylamino, Di-n-propylamino, Diisopropylamino oder Di-n-butylamino.

Analog bedeutet CO-NHA vorzugsweise N-Methylcarbamoyl oder N-Ethylcarbamoyl; CO-NA₂ vorzugsweise N,N-Dimethylcarbamoyl oder N,N-Diethylcarbamoyl und SO₂-A vorzugsweise Methylsulfonyl oder Ethylsulfonyl.

Der Rest Ar bedeutet vorzugsweise unsubstituiertes Phenyl, aber auch ein- oder zweifach substituiertes Phenyl. Falls der Phenylrest zweifach substituiert ist, können die Substituenten gleich oder verschieden sein. Bevorzugte Substituenten an der Phenylgruppe sind F, Cl, Methoxy, CN, CF₃ oder Methyl. Die Substituenten befinden sich im Fall der substituierten Phenylreste in ortho-, meta- und/oder para-Position, wobei zweifach substituierte Phenylreste bevorzugt ortho- und para-substituiert sind. Im einzelnen ist Ar bevorzugt Phenyl, o-, m- oder p-Trifluormethylphenyl, o-, m- oder p-Methoxyphenyl, o-, m- oder p-Fluorphenyl, o-, m- oder p-Methylphenyl, o-, m- oder p-Cyanphenyl oder 2,4-Dimethoxyphenyl, aber auch o-, m- oder p-Ethoxyphenyl, o-, m- oder p-Bromphenyl, 2,3-, 2,5-, 2,6-, 3,4- oder 3,5-Dimethoxyphenyl, 2,3- oder 3,4-Methylendioxyphenyl.

Der Rest B bedeutet einen unsubstituierten oder einfach durch einen der angegebenen Reste substituierten Indol-3-yl- oder Benzimidazol-1-yl-rest. Vorzugsweise sind sie in 5-Stellung, ferner auch in der 4-, 6- oder 7-Stellung substituiert. Bevorzugte Substituenten am Indol-3-ylrest sind CO₂CH₃, CO₂H, CN, CONH₂, CH₂OH, H₂N-CO-NH, CH₃-SO₂-NH und CH₃-CO-NH, aber auch OH, Methoxy, Ethoxy, NH₂ oder NHA, wobei A bevorzugt Methyl oder Ethyl entspricht.

Der Benzimidazolyl-1-rest ist vorzugsweise unsubstituiert, sofern er substituiert ist, sind die gleichen Substituenten besonders bevorzugt, die für den Indol-3-yl-rest angegeben sind.

Der Parameter n kann 1,2,3,4,5 oder 6 sein, vorzugsweise ist er 1, 2 oder 4.

Der Rest Q ist vorzugsweise -(CH₂)₄-, weiterhin -CH₂-, -(CH₂)₂-oder -(CH₂)₃-.

R¹ ist bevorzugt OH, Methoxy oder NH₂, ferner bevorzugt Ethoxy, NH-CH₃ oder N(CH₃)₂.

R² ist vorzugsweise CO-CH₃, CO-NH₂ oder SO₂-CH₃, ferner CO-NH-CH₃ oder CO-N(CH₃)₂, aber auch CO-Phenyl oder SO₂-Methyl.

Dementsprechend sind Gegenstand der Erfindung insbesondere diejenigen Verbindungen der Formel I, in denen mindestens einer der genannten Reste eine der vorstehend angegebenen, insbesondere der vorstehend angegebenen bevorzugten Bedeutungen hat. Einige bevorzugte Gruppen von Verbindungen können durch die folgenden Teilformeln Ia bis Ij ausgedrückt werden, die der Formel I entsprechen und worin die nicht näher bezeichneten Reste und Parameter die bei der Formel I angegebene Bedeutung haben, worin jedoch
- in Ia B: einen in 5-Stellung durch CO-R¹ substituierten Indol-3-yl-rest bedeutet;
- in Ib B: einen in 5-Stellung durch NHR² substituierten Indol-3-yl-rest bedeutet;
- in Ic B: einen in 5-Stellung durch COOH substituierten Indol-3-yl-rest bedeutet;
- in Id B: einen in 5-Stellung durch CCOCH₃ substituierten Indol-3-yl-rest bedeutet;
- in Ie B: einen in 5-Stellung durch CONH₂ substituierten Indol-3-yl-rest bedeutet;
- in If B: einen in 5-Stellung durch CN substituierten Indol-3-yl-rest bedeutet;
- in Ig B: einen in 5-Stellung durch CH₂OH substituierten Indol-3-yl-rest bedeutet;
- in Ih B: einen in 5-Stellung durch OA substituierten Indol-3-yl-rest bedeutet;
- in Ii B: einen unsubstituierten Benzimidazol-1-yl-rest bedeutet;
- in Ij B: einen in 5-Stellung durch CO-R¹ substituierten Benzimidazol-1-yl-rest bedeutet.

Insbesondere sind bevorzugt Verbindungen der Teilformeln Ik sowie Iak bis Iik, die den Teilformeln I sowie Ia bis Ij entsprechen, worin jedoch zusätzlich
- Q: -(CH₂)₄-
bedeutet.

Gegenstand der Erfindung ist ferner ein Verfahren zur Herstellung von 1,4-Benzodioxanderivaten der Formel I sowie von deren Salzen, dadurch gekennzeichnet, daß man eine Verbindung der Formel II

B-Q-X¹ II

worin
- X¹: X oder NH und
- X: Cl, Br, I, OH oder eine reaktionsfähig funktionell abgewandelte OH-Gruppe bedeuten und
- B und Q: die angegebenen Bedeutungen haben,
mit einer Verbindung der Formel III 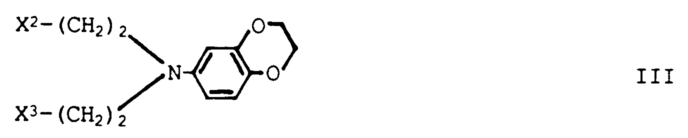
worin
X² und X³ gleich oder verschieden sein können und, falls X¹ = NH₂ ist, jeweils X, andernfalls zusammen NH bedeuten,
umsetzt
oder daß man eine Verbindung der Formel IV

B-Q-N(CH₂-CH₂-X)₂ IV

worin
X, Q und B die angegebenen Bedeutungen haben, mit einer Verbindung der Formel V 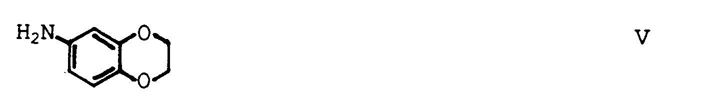
umsetzt
oder daß man eine sonst der Formel I entsprechende Verbindung, die jedoch anstelle der 1,4-Benzodioxangruppe eine 3,4-Dihydroxyphenylgruppe, wobei aber auch die beiden Hydroxygruppen zur Erhöhung der Reaktionsbereitschaft in entsprechend aktivierter Form vorliegen können, mit Ethandiol oder einem entsprechenden reaktiveren Derivat zu einer Verbindung der Formel I umsetzt
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt
und/oder daß man gegebenenfalls eine OA-Gruppe unter Bildung einer OH-Gruppe spaltet und/oder eine Gruppe B in eine andere Gruppe B umwandelt und/oder daß man eine erhaltene Base oder Säure der Formel I durch Behandeln mit einer Säure oder Base in eines ihrer Salze umwandelt.

Die Herstellung der Verbindungen der Formel I erfolgt im übrigen nach an sich bekannten Methoden, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York; DE-OS 33 42 632) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

In den 1,4-Benzodioxanderivaten der Formel II ist X¹ vorzugsweise X; dementsprechend sind in den Verbindungen der Formel III X² und X³ vorzugsweise zusammen NH. Der Rest X ist vorzugsweise Cl oder Br; er kann jedoch auch I, OH oder eine reaktionsfähig funktionell abgewandelte OH-Gruppe bedeuten, insbesondere Alkylsulfonyloxy mit 1-6 (z.B. Methansulfonyloxy) oder Arylsulfonyloxy mit 6-10 C-Atomen (z.B. Benzolsulfonyloxy, p-Toluolsulfonyloxy, 1- oder 2-Naphthalin-sulfonyloxy).

Dementsprechend sind die 1,4-Benzodioxanderivate der Formel I insbesondere durch Umsetzung von Verbindungen der Formel B-Q-Cl oder B-Q-Br mit 6-Piperazino-1,4-benzodioxan (Formel III, worin X² und X³ zusammen eine NH-Gruppe bedeuten; nachstehend als IIIa bezeichnet) erhältlich.

Die Verbindungen der Formeln II und insbesondere III sind zum Teil bekannt; die nicht bekannten Verbindungen der Formeln II und III können leicht analog zu den bekannten Verbindungen hergestellt werden.

Primäre Alkohole der Formel B-Q-OH sind z.B. durch Reduktion der entsprechenden Carbonsäuren oder ihrer Ester erhältlich. Behandeln mit Thionylchlorid, Bromwasserstoff, Phosphortribromid oder ähnlichen Halogenverbindungen liefert die entsprechenden Halogenide der Formel B-Q-Hal. Die entsprechenden Sulfonyloxyverbindungen sind erhältlich aus den Alkoholen B-Q-OH durch Umsetzung mit den entsprechenden Sulfonsäurechloriden.

Die Iodverbindungen der Formel B-Q-I sind z.B. durch Einwirkung von Kaliumiodid auf die zugehörigen p-Toluolsulfonsäureester erhältlich. Die Amine der Formel B-Q-NH₂ sind z.B. aus den Halogeniden mit Phthalimidkalium oder durch Reduktion der entsprechenden Nitrile herstellbar.

Das Piperazinderivat IIIa ist z.B. erhältlich durch Umsetzung von Di-(2-chlorethyl)-amin mit 6-Amino-1,4-benzodioxan. Verbindungen der Formel III (X² und X³ = jeweils X) sind z.B. herstellbar durch Reduktion von 1,4-Benzodioxanen, die in 6-Stellung eine -N(CH₂CO₂A)₂₋Gruppe besitzen, zu den entsprechenden 1,4-Benzodioxanderivaten, die in Position 6 eine -N(CH₂CH₂-OH)₂-Gruppe aufweisen und gegebenenfalls anschließende Umsetzung mit SOCl₂ bzw. PBr₃.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponente B-Q-NH₂ bzw. des Piperazinderivates der Formel IIIa kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Ferner ist es möglich, eine Verbindung der Formel I zu erhalten, indem man eine Verbindung der Formel B-Q-N(CH₂-CH₂-X)₂ (IV) mit 6-Amino-1,4-benzodioxan (V) umsetzt.

Die Verbindungen der Formeln IV sind zum Teil bekannt; die nicht bekannten Verbindungen können leicht in Analogie zu den bekannten hergestellt werden. So lassen sich Verbindungen der Formel IV leicht durch Umsetzung von B-Q-NH₂ mit 1,2-Dihalogenethan, wobei Halogen bevorzugt für Chlor oder Brom steht, herstellen. Ebenso ist es möglich, Verbindungen des Typs IV durch Umsetzung von B-Q-Cl, B-Q-Br oder B-Q-I mit sekundären Aminen der Formel HN(CH₂-CH₂-X)₂ zu erhalten.

Das primäre Amin der Formel V läßt sich beispielsweise ausgehend von Anilin durch die diversen, an sich bekannten Möglichkeiten der elektrophilen Substitution am Aromaten herstellen. Ferner ist es möglich, entsprechend substituierte Nitroverbindungen durch Reduktion in die Amine der Formel V zu überführen.

Die Umsetzung der Verbindungen IV und V verläuft nach Methoden, wie sie für die Alkylierung von Aminen aus der Literatur bekannt sind. Die Komponenten können direkt, ohne Gegenwart eines Lösungsmittels, miteinander verschmolzen werden, gegebenenfalls im geschlossenen Rohr oder im Autoklaven, unter Normaldruck oder unter erhöhtem Druck, wobei ein Inertgas wie z.B. N₂ zur Druckerhöhung zugeführt wird. Es ist äber auch möglich, die Verbindungen in Gegenwart eines inerten Lösungsmittels umzusetzen. Als Lösungsmittel eignen sich die zuvor bei der Umsetzung von II mit III genannten. Ebenso kann sich der Zusatz eines säurebindenden Mittels zur Reaktionsmischung begünstigend auswirken. Es kommen die gleichen Basen, wie zuvor bei der Umsetzung der Verbindungen II und III beschrieben, in Frage.

Die optimale Reaktionszeit liegt, je nach den gewählten Reaktionsbedingungen, zwischen einigen Minuten und 14 Tagen, die Reaktionstemperatur zwischen etwa 0° und 150°, üblicherweise zwischen 20° und 130°.

Eine weitere Möglichkeit Verbindungen der Formel I herzustellen besteht darin, daß man ein Vorprodukt, welches jedoch anstelle der 1,4-Benzodioxangruppe eine 3,4-Dihydroxyphenylgruppe enthält, mit Ethandiol umsetzt. Besonders bevorzugt sind allerdings Varianten dieser Methode, wie sie beispielsweise zur Ether-Darstellung eingesetzt werden, bei denen die Hydroxidgruppen der Reaktionspartner in an sich bekannter Weise aktiviert sind.

Es ist ferner möglich, eine Verbindung der Formel I zu erhalten, indem man ein Vorprodukt, das anstelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z.B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr der oben angeführten Gruppen bzw. zusätzlichen Bindungen enthalten, reduktiv in eine Verbindung der Formel I überzuführen; dabei können gleichzeitig Substituenten in der Gruppe B, die in der Ausgangsverbindung enthalten sind, reduziert werden. Vorzugsweise bedient man sich hierzu des nascierenden Wasserstoffs oder komplexer Metallhydride, ferner der Reduktion nach Wolff-Kishner sowie der Reduktionen mit Wasserstoffgas unter Übergangsmetallkatalyse

Bevorzugte Ausgangsstoffe für die Reduktion entsprechen der Formel VI 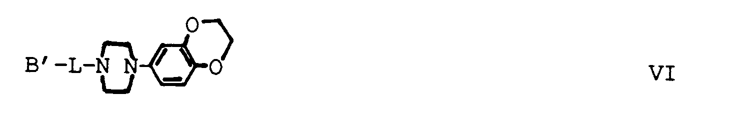
worin
- B': einen Indol-3-yl-rest, der zusätzlich durch eine Arylsulfonylgruppe oder eine Benzylgruppe in 1-Stellung substituiert sein kann oder einem unsubstituierten Benzimidazol-1-yl-rest entspricht
und
- L Q: oder eine dem Rest Q entsprechende Kette, worin jedoch eine oder mehrere -CH₂-Gruppe(n) durch -CO- und/oder ein oder mehrere Wasserstoffatome durch Cl, Br, F, SH- oder OH-Gruppen ersetzt sind,
worin jedoch nicht gleichzeitig B' = B und L = Q sein können.

In den Verbindungen der Formel VI ist L bevorzugt -CO-(CH₂)ₙ₋₂-CO- [im einzelnen -COCO-, -COCH₂CO-, -CO-(CH₂)₂-CO-, -CO-(CH₂)₃-CO-], -(CH₂)ₙ₋₁-CO- [im einzelnen -CH₂-CO-, -CH₂CH₂-CO-, -(CH₂)₃-CO- oder -(CH₂)₄-CO-], ferner z.B. -CO-CH₂CH₂-, -CO-(CH₂)₃-, -CH₂-CO-CH₂CH₂-, -CH₂CH₂-CO-CH₂-, -CO-(CH₂)₄-, -CH₂-CO-(CH₂)3-, -CH₂CH₂-CO-CH₂CH₂- oder -(CH₂)₃-CO-CH₂-.

Verbindungen der Formel VI sind z.B. herstellbar durch Umsetzung von 6-Piperazino-benzo-1,4-dioxan mit einer Verbindung der Formel VII

B'-L-X¹ VII

worin
B', L und X¹ die oben angegebenen Bedeutungen haben, unter den Bedingungen, die zuvor für die Umsetzung von II mit III angegeben sind.

Wird als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden. Geeignet ist auch die Verwendung von Natrium oder einem anderen Alkalimetall ein einem Alkohol wie Ethanol, Isopropanol, Butanol, Amyl- oder Isoamylalkohol oder Phenol. Man kann ferner eine Aluminium-Nickel-Legierung in alkalischwässeriger Lösung, gegebenenfalls unter Zusatz von Ethanol, verwenden. Auch Natrium- oder Aluminiumamalgam in wässerigalkoholischer oder wässeriger Lösung sind zur Erzeugung des nascierenden Wasserstoffs geeignet. Die Umsetzung kann auch in heterogener Phase durchgeführt werden, wobei man zweckmäßig eine wässerige und eine Benzol- oder Toluol-Phase verwendet.

Als Reduktionsmittel können ferner besonders vorteilhaft komplexe Metallhydride, wie LiAlH₄, NaBH₄, Diisobutylaluminiumhydrid oder NaAl(OCH₂CH₂OCH₃)₂H₂ sowie Diboran eingesetzt werden, falls erwünscht unter Zusatz von Katalysatoren wie BF₃, AlCl₃ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit NaBH₄ sind in erster Linie Alkohole wie Methanol oder Ethanol, ferner Wasser sowie wässerige Alkohole als Lösungsmittel geeignet. Nach diesen Methoden reduziert man vorzugsweise bei Temperaturen zwischen -80 und +150°, insbesondere zwischen etwa 0 und etwa 100°.

Besonders vorteilhaft lassen sich -CO-Gruppen in Säureamiden (z.B. solchen der Formel VI, worin L eine -(CH₂)ₙ₋₁-CO-Gruppe bedeutet) mit LiAlH₄ in THF bei Temperaturen zwischen etwa 0 und 66° zu CH₂-Gruppen reduzieren. Dabei können in 1-Stellung des Indolring befindliche Arylsulfonyl-Schutzgruppen gleichzeitig reduktiv abgespalten werden. N-Benzylgruppen können reduktiv mit Natrium in flüssigem Ammoniak abgespalten werden.

Es ist ferner möglich, eine oder mehrere Carbonylgruppen nach der Methode von Wolff-Kishner zu CH₂-Gruppen zu reduzieren, z.B. durch Behandlung mit wasserfreiem Hydrazin in absolutem Ethanol unter Druck bei Temperaturen zwischen etwa 150 und 250°. Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel, wie Diethylenglykol oder Triethylenglykol, in Gegenwart von Alkali, wie Natriumhydroxid, umsetzt. Das Reaktionsgemisch wird in der Regel etwa 3-4 Stunden gekocht. Anschließend wird das Wasser abdestilliert und das gebildete Hydrazon bei Temperaturen bis zu etwa 200° zersetzt. Die Wolff-Kishner-Reduktion kann auch bei Raumtemperatur in Dimethylsulfoxid mit Hydrazin ausgeführt werden.

Darüber hinaus ist es möglich, bestimmte Reduktionen durch Verwendung von H₂-Gas unter katalytischer Wirkung von Übergangsmetallen, wie z.B. Raney-Ni oder Pd durchzuführen. Man kann auf diese Weise z.B. Cl,Br, I, SH oder in bestimmten Fällen auch OH-Gruppen durch Wasserstoff ersetzen. Ebenso können Nitrogruppen durch katalytische Hydrierung mit Pd/H₂ in Methanol oder THF in NH₂-Gruppen umgewandelt werden.

Verbindungen, die sonst der Formel I entsprechen, aber anstelle eines oder mehrerer H-Atome eine oder mehrere solvolysierbare Gruppe(n) enthalten, können zu den Verbindungen der Formel I solvolysiert, insbesondere hydrolysiert werden.

Die Ausgangsstoffe für die Solvolyse sind beispielsweise erhältlich durch Reaktion von IIIa mit Verbindungen, die der Formel II (X¹ = X) entsprechen, aber anstelle eines oder mehrerer H-Atome eine oder mehrere solvolysierbare Gruppe(n) enthalten. So können insbesondere 1-Acylindolderivate (entsprechend der Formel I, aber in 1-Stellung des Ind-Rests eine Acylgruppe enthaltend, vorzugsweise eine Alkanoyl-, Alkylsulfonyl- oder Arylsulfonylgruppe mit jeweils bis zu 10 C-Atomen, wie Methan-, Benzol- oder p-Toluolsulfonyl) zu den entsprechenden in der 1-Stellung des Indolringes unsubstituierten Indolderivaten hydrolysiert werden, z.B. in saurem, besser in neutralem oder alkalischem Medium bei Temperaturen zwischen 0 und 200°. Als Basen verwendet man zweckmäßig Natrium-, Kalium- oder Calciumhydroxid, Natrium- oder Kaliumcarbonat, oder Ammoniak. Als Lösungsmittel wählt man vorzugsweise Wasser; niedere Alkohole wie Methanol, Ethanol; Ether wie THF, Dioxan; Sulfone wie Tetramethylensulfon; oder deren Gemische, besonders die Wasser enthaltenden Gemische. Eine Hydrolyse kann auch bereits beim Behandeln mit Wasser allein erfolgen, insbesondere in der Siedehitze.

Weiterhin kann man eine Verbindung der Formel I nach an sich bekannten Methoden in eine andere Verbindung der Formel I umwandeln.

Verbindungen der Formel I, worin B einen durch CO-R¹ substituierten Benzimidazol-1-yl- oder Indol-3-yl-rest bedeutet, können durch Derivatisierung entsprechender Carboxy-benzimidazol-1-yl- oder Carboxy-indol-3-yl-Verbindungen erhalten werden. Man kann z.B. die Säuren oder ihre reaktionsfähigen Derivate, wie z.B. ihre Säurehalogenide oder Anhydride mit entsprechenden Alkoholen oder Alkoholaten, unter Verwendung der an sich bekannten Methodik oder einer der zahlreichen Varianten, verestern. Ferner ist es möglich, Säuren, Säurehalogenide, Anhydride oder Ester mit primären oder sekundären, alipha- tischen oder cyclischen Aminen zu amidieren. Bevorzugt ist die Umsetzung der freien Carbonsäure mit dem Amin unter den Bedingungen einer Peptidsynthese. Diese Reaktion gelingt vorzugsweise in Gegenwart eines Dehydratisierungsmittels, z.B. eines Carbodiimids wie Dicyclohexylcarbodiimid oder N-(3-Dimethylaminopropyl)-N-ethyl-carbodiimid, ferner Propanphosphonsäurean- hydrid (vgl. Angew. Chem. 92, 129 (1980)), Diphenylphosphoryl azid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40, vorzugsweise zwischen 0 und 30°. Anstelle der Säure bzw. des Amids können auch reaktionsfähige Derivate dieser Stoffe in die Reaktion eingesetzt werden, z.B. solche, in denen reaktive Gruppen intermediär durch Schutzgruppen blockiert sind. Die Säuren können auch in Form ihrer aktivierten Ester verwendet werden, die zweckmäßig in situ gebildet werden, z.B. durch Zusatz von 1-Hydroxybenztriazol oder N-Hydroxysuccinimid.

Weiterhin kann man cyan-substituierte Reste B zu Carboxy-indol-3-yl- oder Carboxybenzimidazol-1-yl-resten oder Carbamido-indol-3-yl- bzw. Carbamidobenzimidazol-1-yl-resten hydrolysieren.

Verbindungen der Formel I, die durch O-Alkyl substituiert sind, können durch Etherspaltung in die entsprechenden Hydroxyderivate überführt werden. Z.B. kann man die Ether spalten durch Behandeln mit Dimethylsulfid-Bortribromid-Komplex, z.B. in Toluol, Ethern wie THF oder Dimethylsulfoxid, oder durch Verschmelzen mit Pyridin- oder Anilin-hydrohalogeniden, vorzugsweise Pyridinhydrochlorid, bei etwa 150-250°.

Die Verbindungen der Formel I können ein oder mehrere Asymmetriezentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr Asymmetriezentren auf, dann fallen sie bei der Synthese im allgemeinen als Gemische von Racematen an, aus denen man die einzelnen Racemate, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre optischen Antipoden getrennt werden. Vorzugsweise werden aus dem Racemat durch Umsetzung mit einem optisch aktiven Trennmittel Diastereomere gebildet. Als Trennmittel eignen sich z.B. optisch aktive Säuren, wie die D- und L-Formen von Weinsäuren, Dibenzoylweinsäure, Diacetylweinsäure, Camphersulfonsäuren, Mandelsäure, Äpfelsäure oder Milchsäure. Die verschiedenen Formen der Diastereomeren können in an sich bekannter Weise, z.B. durch fraktionierte Kristallisation, getrennt, und die optisch aktiven Verbindungen der Formel I können in an sich bekannter Weise aus den Diastereomeren in Freiheit gesetzt werden.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure,Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden, sofern keine weiteren aciden Gruppen im Molekül vorliegen. In jenen Fällen, wo die Verbindungen der Formel I über freie Säuregruppen verfügen, kann durch Behandlung mit Basen ebenfalls eine Salzbildung erreicht werden. Als Basen eignen sich Alkalimetallhydroxide, Erdalkalimetallhydroxide oder organische Basen in Form von primären, sekundären oder tertiären Aminen.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoffe(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- und Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der therapeutischen Behandlung des menschlichen oder tierischen Körpers und bei der Bekämpfung von Krankheiten verwendet werden. Sie eignen sich zur Behandlung von Erkrankungen des Zentralnervensystems wie Spannungszuständen, Depressionen und/oder Psychosen und von Nebenwirkungen bei der Behandlung der Hypertonie (z.B. mit α-Methyldopa). Ferner können die Verbindungen in der Endokrinologie und Gynäkologie Verwendung finden, z.B. zur Therapie von Akromegalie, Hypogonadismus, sekundärer Amenorrhoe, prämenstruellem Syndrom, unerwünschter puerperaler Laktation, weiterhin zur Prophylaxe und Therapie cerebraler Störungen (z.B. Migräne), insbesondere in der Geriatrie ähnlich wie gewisse Ergot-Alkaloide und zur Bekämpfung der Folgen cerebraler Infarktgeschehen (Apoplexia cerebri), wie Schlaganfall und cerebraler Ischämien.

Dabei werden die erfindungsgemäßen Substanzen in der Regel in Analogie zu bekannten, im Handel befindlichen Präparaten (z.B. Bromocriptin, Dihydroergocornin) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Temperaturen sind in °C angegeben. Rf-Werte wurden dünnschichtchromatographisch an Kieselgel erhalten.

### Beispiel 1

Man rührt eine Lösung von 3,6 g 3-(4-Chlorbutyl)-5-methoxycarbonyl-indol [erhältlich durch Umsetzung von 5-Methoxycarbonyl-indol mit 4-Chlorbutyrylchlorid zu 3-(4-Chlorbutyryl)-5-methoxy-indol und anschließender Reduktion mit Diboran zu 3-(4-Chlorbutyl)-5-methoxycarbonyl-indol] und 3,4 g 6-Piperazino-1,4-benzodioxan ("A") in 200 ml Acetonitril 14 Std. bei Raumtemperatur, arbeitet wie üblich auf und erhält 6-[4-(4-(5-Methoxycarbonyl-indol-3-yl)-butyl)-piperazino]-1,4-benzodioxan, F. 177-179° (Dihydrochlorid).

Analog erhält man durch Umsetzung von "A"
- mit: 3-(4-Chlorbutyl)-5-fluor-indol 6-[4-(4-(5-Fluor-indol-3-yl)-butyl)-piperazino]-1,4-benzodioxan, F. 227-228 °C (Hydrochlorid);
- mit: 3-(4-Brombutyl)-5-brom-indol 6-[4-(4-(5-Brom-indol-3-yl)-butyl)-piperazino]-1,4-benzodioxan;
- mit: 3-(4-Chlorbutyl)-5-cyan-indol 6-[4-(4-(5-Cyan-indol-3-yl)-butyl)-piperazino]-1,4-benzodioxan;
- mit: 3-(4-Chlorbutyl)-5-chlor-indol 6-[4-(4-(5-Chlor-indol-3-yl)-butyl)-piperazino]-1,4-benzodioxan, F. 205-207 ° (Hydrochlorid);
- mit: 3-(4-Chlorbutyl)-5-indol-carbonsäure 6-[4-(4-(5-Carboxy-indol-3-yl)-butyl)-piperazino]-1,4-benzodioxan, F. 241-243 ° (Hydrochlorid);
- mit: 3-(4-Chlorbutyl)-6-indol-carbonsäuremethylester 6-[4-(4-(6-Methoxycarbonyl-indol-3-yl)-butyl)-piperazino]-1,4-benzodioxan;
- mit: 3-(3-Chlorpropyl)-5-fluor-indol 6-[4-(3-(5-Fluor-indol-3-yl)-propyl)-piperazino]-1,4-benzodioxan;
- mit: 3-(3-Brompropyl)-5-brom-indol 6-[4-(3-(5-Brom-indol-3yl)-propyl)-piperazino]-1,4-benzodioxan;
- mit: 3-(3-Chlorpropyl)-5-cyan-indol 6-[4-(3-(5-Cyan-indol-3-yl)-propyl)-piperazino]-1,4-benzodioxan;
- mit: 3-(3-Chlorpropyl)-6-cyan-indol 6-[4-(3-(6-Cyan-indol-3-yl)-propyl)-piperazino]-1,4-benzodioxan;
- mit: 3-(4-Chlorobutyl)-5-methoxy-indol 6-[4-(4-(5-Methoxy-indol-3-yl)-butyl)-piperazino]-1,4-benzodioxan, F. 107-109°;
- mit: 3-(4-Chlorobutyl)-5-hydroxy-indol 6-[4-(4-(5-Hydroxy-indol-3-yl)-butyl)-piperazino]-1,4-benzodioxane, F. 212-214°;
- mit: 3-(3-Chlorpropyl)-indol-5-carbonsäure 6-[4-(3-(5-Carboxy-indol-3-yl)-propyl)-piperazino]-1,4-benzodioxan;
- mit: 3-(2-Chlorethyl)-5-fluor-indol 6-[4-(2-(5-Fluor-indol-3-yl)-ethyl)-piperazino]-1,4-benzodioxan.

### Beispiel 2

Analog Beispiel 1 erhält man durch Umsetzung von 1-(4-Brom-butyl)-benzimidazol mit 6-Piperazino-1,4-benzodioxan ("A") in 200 ml Acetonitril 6-[4-(4-(Benzimidazol-1-yl)-butyl)-piperazino]-1,4-benzodioxan, F. 247-248°.

Analog erhält man durch Umsetzung von "A"
- mit: 1-(4-Chlorbutyl)-5-fluor-benzimidazol 6-[4-(4-(5-Fluor-benzimidazol-1-yl)-butyl)-piperazino]-1,4-benzodioxan;
- mit: 1-(4-Brombutyl)-5-brom-benzimidazol 6-[4-(4-(5-Brom-benzimidazol-1-yl)-butyl)-piperazino]-1,4-benzodioxan;
- mit: 1-(4-Chlorbutyl)-5-cyan-benzimidazol 6-[4-(4-(5-Cyan-benzimidazol-1-yl)-butyl)-piperazino]-1,4-benzodioxan;
- mit: 1-(4-Chlorbutyl)-5-benzimidazol-carbonsäureamid 6-[4-(4-(5-Carbamoyl-benzimidazol-1-yl)-butyl)-piperazino]-1,4-benzodioxan;
- mit: 1-(4-Chlorbutyl)-5-benzimidazol-carbonsäure 6-[4-(4-(5-Carboxy-benzimidazol-1-yl)-butyl)-piperazino]-1,4-benzodioxan;
- mit: 1-(4-Chlorbutyl)-5-benzimidazol-carbonsäuremethylester 6-[4-(4-(5-Methylcarboxy-benzimidazol-1-yl)-butyl)-piperazino]-1,4-benzodioxan;
- mit: 1-(3-Chlorpropyl)-5-fluor-benzimidazol 6-[4-(3-(5-Fluor-benzimidazol-1-yl)-propyl)-piperazino]-1,4-benzodioxan;
- mit: 1-(3-Brompropyl)-5-brom-benzimidazol 6-[4-(3-(5-Brom-benzimidazol-1-yl)-propyl)-piperazino]-1,4-benzodioxan;
- mit: 1-(3-Chlorpropyl)-5-cyan-benzimidazol 6-[4-(3-(5-Cyan-benzimidazol-1-yl)-propyl)-piperazino]-1,4-benzodioxan;
- mit: 1-(3-Chlorpropyl)-5-benzimidazol-carbonsäureamid 6-[4-(3-(5-Carbamoyl-benzimidazol-1-yl)-propyl)-piperazino]-1,4-benzodioxan;
- mit: 1-(3-Chlorpropyl)-5-benzimidazol-carbonsäure 6-[4-(3-(5-Carboxy-benzimidazol-1-yl)-propyl)-piperazino]-1,4-benzodioxan;
- mit: 1-(2-Chlorethyl)-5-fluor-benzimidazol 6-[4-(2-(5-Fluor-benzimidazol-1-yl)-ethyl)-piperazino]-1,4-benzodioxan;
- mit: 1-(2-Bromethyl)-5-brom-benzimidazol 6-[4-(2-(5-Brom-benzimidazol-1-yl)-ethyl)-piperazino]-1,4-benzodioxan;
- mit: 1-(2-Chlorethyl)-5-cyan-benzimidazol 6-[4-(2-(5-Cyan-benzimidazol-1-yl)-ethyl)-piperazino]-1,4-benzodioxan;
- mit: 1-(2-Chlorethyl)-5-benzimidazol-carbonsäureamid 6-[4-(2-(5-Carbamoyl-benzimidazol-1-yl)-ethyl)-piperazino]-1,4-benzodioxan;
- mit: 1-(2-Chlorethyl)-5-benzimidazol-carbonsäure 6-[4-(2-(5-Carboxy-benzimidazol-1-yl)-ethyl)-piperazino]-1,4-benzodioxan.

### Beispiel 3

Ein Gemisch von 2,18 g 3-(4-Aminobutyl)-5-ethoxycarbonyl-indol [erhältlich aus 5-Ethoxycarbonyl-indol durch Umsetzung mit 4-Chlorbutyrylchlorid, Reduktion des Produktes zu 3-(4-Chlorbutyl)-5-ethoxycarbonyl-indol und Überführung in 3-(4-Phthal-imido-butyl)-5-ethoxycarbonyl-indol] und einem Äquivalent 6-(N,N-Bis-(2-chlorethyl)-amino)-1,4-benzodioxan ("B") in 40 ml Aceton und 40 ml Wasser wird 24 Std. gekocht und wie üblich aufgearbeitet. Man erhält 6-[4-(4-(5-Ethoxycarbonyl-indol-3-yl)-butyl)-piperazino]-1,4-benzodioxan.

Analog erhält man durch Umsetzung von "B"
- mit: 3-(4-Aminobutyl)-5-N-methyl-carbamoyl-indol 6-[4-(4-(5-N-Methyl-carbamoyl-indol-3-yl)-butyl)-piperazino]-1,4-benzodioxan;
- mit: 3-(4-Aminobutyl)-5-N,N-dimethyl-carbamoyl-indol 6-[4-(4-(5-N,N-dimethyl-carbamoyl-indol-3-yl)-butyl)-piperazino]-1,4-benzodioxan;
- mit: 3-(4-Aminobutyl)-6-cyan-indol 6-[4-(4-(6-Cyan-indol-3-yl)-butyl)-piperazino]-1,4-benzodioxan;
- mit: 3-(4-Aminobutyl)-6-brom-indol 6-[4-(4-(6-Brom-indol-3-yl)-butyl)-piperazino]-1,4-benzodioxan;
- mit: 3-(4-Aminobutyl)-5-indolyl-N-methyl-harnstoff 6-[4-(4-(5-N-Methyl-ureido-indol-3-yl)-butyl)-piperazino]-1,4-benzodioxan;
- mit: 3-(4-Aminobutyl)-5-indolyl-N,N-dimethyl-harnstoff 6-[4-(4-(5-N,N-Dimethyl-ureido-indol-3yl)-butyl)-piperazino]-1,4-benzodioxan;
- mit: 3-(3-Aminopropyl)-5-methoxy-indol 6-[4-(3-(5-Methoxy-indol-3-yl)-propyl)-piperazino]-1,4-benzodioxan;
- mit: 3-(3-Aminopropyl)-6-brom-indol 6-[4-(3-(6-Brom-indol-3-yl)-propyl)-piperazino]-1,4-benzodioxan;
- mit: 3-(3-Aminopropyl)-6-methoxy-indol 6-[4-(3-(6-Methoxy-indol-3-yl)-propyl)-piperazino]-1,4-benzodioxan;
- mit: 3-(3-Aminopropyl)-4-methoxy-indol 6-[4-(3-(4-Methoxy-indol-3-yl)-propyl)-piperazino]-1,4-benzodioxan;
- mit: 3-(3-Aminopropyl)-4-cyan-indol 6-[4-(3-(4-Cyan-indol-3-yl)-propyl)-piperazino]-1,4-benzodioxan;
- mit: 3-(3-Aninopropyl)-5-ethoxy-indol 6-[4-(3-(5-Ethoxy-indol-3-yl)-propyl)-piperazino]-1,4-benzodioxan;
- mit: 3-(3-Aminopropyl)-indol-6-carbonsäuremethylester 6-[4-(3-(6-Methoxycarbonyl-indol-3-yl)-propyl)-piperazino]-1,4-benzodioxan;
- mit: 3-(4-Aminobutyl)-5-N-(3-chinuclidinyl)-carbamoyl-indol 6-[4-(4-(5-N-(3-Chinuclidinyl)-carbamoyl-indol-3-yl)-butyl)-piperazino]-1,4-benzodioxan, F. 212-219°;
- mit: 3-(4-Aminobutyl)-5-N-[(3-pyridinyl)-methyl]-carbamoyl-indol 6-[4-(4-(5-N-(3-Pyridinyl)-methyl-carbamoyl-indol-3-yl)-butyl)-piperazino]-1,4-benzodioxan, F. 150-152°;
- mit: 3-(4-Aminobutyl)-indol-5-carbonsäurepiperazid 6-[4-(4-(5-Piperazino-carbonyl-indol-3-yl)-butyl)-piperazino]-1,4-benzodioxan F. 164-166°;
- mit: 3-(4-Aminobutyl)-5-N-(N',N''-di-carbethoxy-pyrazolidin-4-yl)-carbamoyl-indol 6-[4-(4-(5-N-(N',N''-Di-carbethoxy-pyrazolidin-4-yl)-carbamoyl-indol-3-yl)-butyl)-piperazino]-1,4-benzodioxan, F.218-222°;
- mit: 3-(2-Aminoethyl)-4-fluor-indol 6-[4-(2-(4-Fluor-indol-3-yl)-ethyl)-piperazino]-1,4-benzodioxan.

### Beispiel 4

Analog Beispiel 3 erhält man durch Umsetzung von 1-(4-Aminobutyl)-5-ethoxycarbonyl-benzimidazol mit 6-(N,N-Bis-(2-chlorethyl)-amino)-1,4-benzodioxan ("B") 6-[4-(4-(5-Ethoxycarbonylbenzimidazol-1-yl)-butyl)-piperazino]-1,4-benzodioxan.

Analog erhält man durch Umsetzung von "B"
- mit: 1-(4-Aminobutyl)-5-N-methyl-carbamoyl-benzimidazol 6-[4-(4-(5-N-Methyl-carbamoyl-benzimidazol-1-yl)-butyl-piperazino]-1,4-benzodioxan;
- mit: 1-(4-Aminobutyl)-5-N,N-dimethyl-carbamoyl-benzimidazol 6-[4-(4-(5-N,N-dimethyl-carbamyl-benzimidazol-1-yl)-butyl)-piperazino]-1,4-benzodioxan;
- mit: 1-(4-Aminobutyl)-6-cyan-benzimidazol 6-[4-(4-(6-Cyan-benzimidazol-1-yl)-butyl)-piperazino]-1,4-benzodioxan;
- mit: 1-(4-Aminobutyl)-6-brom-benzimidazol 6-[4-(4-(6-Brom-benzimidazol-1-yl)-butyl)-piperazino]-1,4-benzodioxan;
- mit: 1-(4-Aminobutyl)-5-benzimidazolyl-N-methyl-harnstoff 6-[4-(4-(5-N-Methyl-ureido-benzimidazol-1-yl)-butyl)-piperazino]-1,4-benzodioxan;
- mit: 1-(4-Aminobutyl)-5-benzimidazolyl-N,N-dimethyl-harnstoff 6-[4-(4-(5-N,N-Dimethyl-ureido-benzimidazol-1-yl)-butyl)-piperazino]-1,4-benzodioxan;
- mit: 1-(3-Aminopropyl)-5-methoxy-benzimidazol 6-[4-(3-(5-Methoxy-benzimidazol-1-yl)-propyl)-piperazino]-1,4-benzodioxan;
- mit: 1-(3-Aminopropyl)-6-brom-benzimidazol 6-[4-(3-(6-Brom-benzimidazol-1-yl)-propyl)-piperazino]-1,4-benzodioxan;
- mit: 1-(3-Aminopropyl)-6-methoxy-benzimidazol 6-[4-(3-(6-Methoxy-benzimidazol-1-yl)-propyl)-piperazino]-1,4-benzodioxan;
- mit: 1-(3-Aminopropyl)-4-methoxy-benzimidazol 6-[4-(3-Methoxy-benzimidazol-1-yl)-propyl)-piperazino]-1,4-benzodioxan;
- mit: 1-(3-Aminopropyl)-4-cyan-benzimidazol 6-[4-(3-(4-Cyan-benzimidazol-1-yl)-propyl)-piperazino]-1,4-benzodioxan;
- mit: 1-(3-Aminopropyl)-5-ethoxy-benzimidazol 6-[4-(3-(5-Ethoxy-benzimidazol-1-yl)-propyl)-piperazino]-1,4-benzodioxan;
- mit: 1-(3-Aminopropyl)-6-benzimidazol-carbonsäuremethylester 6-[4-(3-(6-Methoxy-carbonyl-benzimidazol-1-yl)-propyl)-piperazino]-1,4-benzodioxan;
- mit: 1-(2-Aminoethyl)-4-fluor-benzimidazol 6-[4-(2-(4-Fluor-benzimidazol-1-yl)-ethyl)-piperazino]-1,4-benzodioxan.

### Beispiel 5

Analog Beispiel 1 erhalt man durch Umsetzung von 3-(4-Chlor-butyl)-5-nitro-indol mit "A" 6-[4-(4-(5-Nitro-indol-3-yl)-butyl)-piperazino]-1,4-benzodioxan.

### Beispiel 6

Eine Lösung von 4,21 g 6-[4-(4-(5-Amino-indol-3-yl)butyl)-piperazino]-1,4-benzodioxan ("C") [erhältlich gemäß Beispiel 5] in 35 ml THF wird mit einer Lösung von 0,9 g Acetylchlorid in 10 ml THF versetzt, 2 Std. bei 50° gerührt, eingedampft und wie üblich aufgearbeitet. Man erhält 6-[4-(4-(5-Acetamido-indol-3-yl)-butyl)-piperazino]-1,4-benzodioxan.

Analog erhält man durch Umsetzung von "C"
- mit: Benzoylchlorid 6-[4-(4-(5-Benzamido-indol-3-yl)-butyl)-piperazino]-1,4-benzodioxan;
- mit: Methansulfonylchlorid 6-[4-(4-(5-Methansulfonylamino-indol-3-yl)-butyl)-piperazino]-1,4-benzodioxan
- mit: N,N-Dimethylcarbamoylchlorid 6-[4-(4-(5-N,N-dimethyl-ureido-indol-3-yl)-butyl)-piperazino]-1,4-benzodioxan;
- mit: N,N-Diethylcarbamoylchlorid 6-[4-(4-(5-N,N-diethyl-ureido-indol-3-yl)-butyl)-piperazino]-1,4-benzodioxan.

### Beispiel 7

Analog Beispiel 6 erhält man durch Umsetzung von 6-[4-(4-(6-Aminobenzimidazol-1-yl)-butyl)-piperazino]-1,4-benzodioxan ("D") mit Acetylchlorid das 6-[4-(4-(6-Acetamido-benzimidazol-1-yl)-butyl)-piperazino]-1,4-benzodioxan.

Analog erhält man durch Umsetzung von "D"
- mit: Benzoylchlorid 6-[4-(4-(6-Benzamido-benzimidazol-1-yl)-butyl)-piperazino]-1,4-benzodioxan;
- mit: Methansulfonylchlorid 6-[4-(4-(6-Mthansulfonylamido-benzimidazol-1-yl)-butyl)-piperazino]-1,4-benzodioxan;
- mit: N,N-Dimethylcarbamoylchlorid 6-[4-(4-(6-N,N-dimethyl-ureido-benzimidazol-1-yl)-butyl-piperazino]-1,4-benzodioxan;
- mit: N,N-Diethylcarbamoylchlorid 6-[4-(4-(6-N,N-diethyl-ureido-benzimidazol-1-yl)-butyl-piperazino]-1,4-benzodioxan.

### Beispiel 8

Analog Beispiel 7 erhält man durch Umsetzung von 6-[4-(4-(-Aminobenzimidazol-1-yl)-butyl)-piperazino]-1,4-benzodioxan ("E") mit Acetylchlorid das 6-[4-(4-(5-Acetamido-benzimidazol-1-yl)-butyl)-piperazino]-1,4-benzodioxan.

Analog erhält man durch Umsetzung von "E"
- mit: Benzoylchlorid 6-[4-(4-(5-Benzamido-benzimidazol-1-yl)-butyl)-piperazino]-1,4-benzodioxan;
- mit: Methansulfonylchlorid 6-[4-(4-(5-Methansulfonylamino-benzimidazol-1-yl)-butyl)-piperazino]-1,4-benzodioxan;
- mit: N,N-Dimethylcarbamoylchlorid 6-[4-(4-(5-N,N-dimethyl-ureido-benzimidazol-1-yl)-butyl)-piperazino]-1,4-benzodioxan;
- mit: N,N-Diethylcarbamoylchlorid 6-[4-(4-(5-N,N-diethyl-ureido-benzimidazol-1-yl)-butyl)-piperazino]-1,4-benzodioxan.

### Beispiel 9

Eine Suspension von 3,8 g 6-[4-(4-(5-Nitro-indol-3-yl)-butyl)-piperazino]-1,4-benzodioxan in 45 ml Methanol wird unter Rühren an 0,1 %iger Pd-C bei 20° und 1 bar bis zum Ende der H₂-Aufnahme hydriert. Man gießt auf Eiswasser, arbeitet wie üblich auf und erhält 6-[4-(4-(5-Amino-indol-3-yl)-butyl)-piperazino]-1,4-benzodioxan.

Analog erhält man
- aus: 6-[4-(4-(4-Nitro-indol-3-yl)-butyl)-piperazino]-1,4-benzodioxan 6-[4-(4-(4-Amino-indol-3-yl)-butyl)-piperazino]-1,4-benzodioxan;
- aus: 6-[4-(4-(6-Nitro-indol-3-yl)-butyl)-piperazino)-1,4-benzodioxan 6-[4-(4-(6-Amino-indol-3-yl)-butyl)-piperazino]-1,4-benzodioxan;
- aus: 6-[4-(4-(5-Nitro-benzimidazol-1-yl)-butyl)-piperazino)-1,4-benzodioxan 6-[4-(4-(5-Amino-benzimidazol-1-yl)-butyl)-piperazino]-1,4-benzodioxan;
- aus: 6-[4-(4-(4-Nitro-benzimidazol-1-yl)-butyl)-piperazino]-1,4-benzodioxan 6-[4-(4-(4-Amino-benzimidazol-1-yl)-butyl)-piperazino]-1,4-benzodioxan.

### Beispiel 10

Eine Mischung von 4,16 g 6-[4-(4-(5-Cyan-benzimidazol-1-yl)-butyl)-piperazino]-1,4-benzodioxan, 2,4 g NaOH, 50 ml H₂O und 40 ml Diethylenglykolmonoethylether wird 3 Std. bei 140° Badtemperatur gerührt. Anschließend kühlt man auf Raumtemperatur ab, arbeitet wie üblich auf und erhält 6-[4-(4-(5-Carbamoyl-benzimidazol-1-yl)-butyl)-piperazino]-1,4-benzodioxan.

Analog erhält man durch partielle Hydrolyse der entsprechenden Nitrile:
6-[4-(4-(6-Carbamoyl-benzimidazol-1-yl)-butyl)-piperazino]-1,4-benzodioxan;
6-[4-(4-(5-Carbamoyl-indol-3yl)-butyl)-piperazino]-1,4-benzodioxan;
6-[4-(4-(6-Carbamoyl-indol-3-yl)-butyl)-piperazino]-1,4-benzodioxan.

### Beispiel 11

Zu einer Suspension von 0,6 g Lithiumaluminiumhydrid in 20 ml THF wird unter Rühren in einer N₂-Atmosphäre bei 20° eine Lösung von 4,4 g 6-[4-(4-(5-Methoxycarbonyl-benzimidazol-1-yl)-butyl)-piperazino]-1,4-benzodioxan in 40 ml THF zugetropft. Man rührt 1 Std. bei 20°, zersetzt mit verdünnter Natronlauge, filtriert, arbeitet das Filtrat wie üblich auf und erhält 6-[4-(4-(5-Hydroxymethyl-benzimidazol-1-yl)-butyl)-piperazino]-1,4-benzodioxan.

### Beispiel 12

In eine siedende Lösung von 3,1 g 6-[4-(4-(5-Carboxyl-indol-3-yl)-butyl)-piperazino]-1,4-benzodioxan in 50 ml absolutem Methanol wird 2 Std. HCl-Gas eingeleitet. Anschließend kocht man eine weitere Stunde, arbeitet wie üblich auf und erhält 6-[4-(4-(5-Methoxycarbonyl-indol-3-yl)-butyl)-piperazino]-1,4-benzodioxan. (Dihydrochlorid), F . 177-179°.

### Beispiel 13

In eine siedene Lösung von 3,1 g 6-[4-(4-(5-Carboxyl-benzimidazol-1-yl)-butyl)-piperazino]-1,4-benzodioxan in 50 ml absolutem Methanol wird 2 Std. HCl-Gas eingeleitet. Anschließend kocht man eine weitere Stunde, arbeitet wie üblich auf und erhält 6-[4-(4-(5-Methoxycarbonyl-benzimidazol-1-yl)-butyl)-piperazino]-1,4-benzodioxan.

### Beispiel 14

Man kocht 4,7 g 6-[4-(4-(5-Methoxycarbonyl-indol-3-yl)-butyl)-piperazino]-1,4-benzodioxan 0,5 Std. mit 100 ml 2n ethanolischer KOH, arbeitet wie üblich auf und erhält 6-[4-(4-(5-Carboxy-indol-3-yl)-butyl)-piperazino]-1,4-benzodioxan, F. 241-243° (Hydrochlorid).

### Beispiel 15

Man rührt eine Lösung von 7,4 g 3-[4-(N,N-Bis-(2-chlorethyl)-aminobutyl)-5-ethoxy-indol und einem Äquivalent 6-Amino-1,4-benzodioxan in 200 ml Acetonitril über eine Zeitdauer von 12 Std. bei Raumtemperatur, arbeitet wie üblich auf und erhält 6-[4-(4-(5-Ethoxy-indol-3-yl)-butyl)-piperazino]-1,4-benzodioxan.

Analog erhält man durch Umsetzung von 6-Amino-1,4-benzodioxan
- mit: 3-[4-(N,N-Bis-(2-chlorethyl)-amino-butyl)-4-ethoxy-indol 6-[4-(4-(4-Ethoxy-indol-3-yl)-butyl)-piperazino]-1,4-benzodioxan;
- mit: 1-[4-(N,N-Bis-(2-chlorethyl)-amino-butyl)-5-ethoxy-benzimidazol 6-[4-(4-(5-Ethoxy-benzimidazol-1-yl)-butyl)-piperazino]-1,4-benzodioxan;
- mit: 1-[4-(N,N-Bis-(2-chlorethyl)-amino-butyl)-6-ethoxy-benzimidazol 6-[4-(4-(6-Ethoxy-benzimidazol-1-yl)-butyl)-piperazino]-1,4-benzodioxan;
- mit: 1-[3-(N,N-Bis-(2-chlorethyl)-amino-propyl)-5-ethoxy-benzimidazol 6-[4-(3-(5-Ethoxy-benzimidazol-1-yl)-propyl)-piperazino]-1,4-benzodioxan;
- mit: 3-[2-(N,N-Bis-(2-chlorethyl)-amino-ethyl)-4-ethoxy-indol 6-[4-(2-(4-Ethoxy-indol-3-yl)-butyl)-piperazino]-1,4-benzodioxan;
- mit: 3-[2-(N,N-Bis-(2-chlorethyl)-amino-ethyl)-5-methoxy-indol 6-[4-(2-(5-Methoxy-indol-3-yl)-butyl)-piperazino]-1,4-benzodioxan.

### Beispiel 16

Man kocht 4,7 g 6-[4-(4-(1-Benzolsulfonyl-5-brom-indol-3-yl)-butyl)-piperazino]-1,4-benzodioxan mit 1,5 g KOH in wäßriger Ethanollösung über 16 Std., arbeitet wie üblich auf und erhält 6-[4-(4-(5-Bromindol-3-yl)-butyl)-piperazino]-1,4-benzodioxan.

### Beispiel 17

Analog Beispiel 9 erhält man durch katalytische Reduktion (Pd-C/H₂)
- aus: 6-[4-(4-(6-Nitro-benzimidazol-1-yl)-butyl)-piperazino]-1,4-benzodioxan 6-[4-(4-(6-Amino-benzimidazol-1-yl)-butyl)-piperazino]-1,4-benzodioxan
- aus: 6-[4-(4-(7-Nitro-benzimidazol-1-yl)-butyl)-piperazino]-1,4-benzodioxan 6-[4-(4-(7-Amino-benzimidazol-1-yl)-butyl)-piperazino]-1,4-benzodioxan.

### Beispiel 18

Eine Mischung von 3,1 g 6-[4-(3-(5-Cyan-indol-3-yl)-propyl)-piperazino]-1,4-benzodioxan, 2,7 g NaOH, 100 ml Wasser und 50 ml Diethylenglykolmonoethylether wird 3 Std. bei 140 ° Badtemperatur gerührt. Man kühlt ab, arbeitet wie üblich auf und erhält 6-4-(3-(5-Carbamoyl-indol-3-yl)-propyl)-piperazino]-1,4-benzodioxan.

Analog erhält man durch partielle Hydrolyse der entsprechenden Cyanindole:
6-[4-(4-(5-Carbamoyl-indol-3-yl)-butyl)-piperazino]-1,4-benzodioxan, F. 215° (Zers.);
6-[4-(4-(6-Carbamoyl-indol-3-yl)-butyl)-piperazino]-1,4-benzodioxan;
6-[4-(4-(7-Carbamoyl-indol-3-yl)-butyl)-piperazino]-1,4-benzodioxan.

Die nachstehenden Beispiele betreffen pharmazeutische Zubereitungen, die Amine der Formel I oder ihre Säureadditionssalze enthalten:

### Beispiel A: Tabletten

Ein Gemisch von 1 kg 6-[4-(4-Benzimidazol-1-yl)-butyl-piperazino]-1,4-benzodioxan, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel B: Dragees

Analog Beispiel A werden Tabletten gepreßt, die anschließend in üblicher Weise mit einerm Überzug aus Saccharose, Kartoffelstärke, Talk, Tragant und Farbstoff überzogen werden.

### Beispiel C: Kapseln

2 kg 6-[4-(4-(5-Methoxy-indol-3-yl)-butyl)-piperazino]-1,4-benzodioxan werden in üblicher Weise in Hartgelatinekapseln gefüllt, so daß jede Kapsel 20 mg des Wirkstoffs enthält.

### Beispiel D: Ampullen

Eine Lösung von 1 kg 6-[4-(4-(5-Methoxy-indol-3-yl)-butyl)-piperazino]-1,4-benzodioxan in 60 l zweifach destilliertem Wasser wird steril filtriert, in Ampul- len abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

## Patentansprüche

1. 1,4-Benzodioxanderivate der Formel I 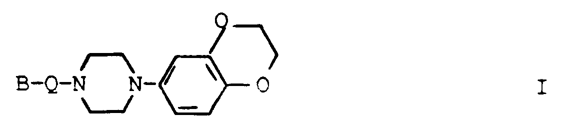 worin
B einen unsubstituierten oder einfach durch CN, CO-R¹, CₙH₂ₙ-R¹, Hal, OH, OA, O-CₙH₂ₙ-CO-R¹, oder NHR² substituierten Indol-3-yl- oder Benzimidazol-1-yl-rest,
R¹ OH, OA, NH₂, NHA, NA₂_{,} NH-3-chinuclidinyl, NH-CH₂-3-pyridinyl, NH-1-piperazinyl oder NH-4-(N',N''-di-carbethoxy-pyrazolidinyl),
R² H, A, CO-A, CO-Ar, CO-NH₂, CO-NHA, CO-NA₂, SO₂-Ar oder SO₂-A,
Q C₂H₂ₙ,
n 1, 2, 3, 4, 5 oder 6,
A Alkyl mit 1-6 C-Atomen,
Ar einen unsubstituierten oder einen ein- oder zweifach durch A, Hal, CN, OH und/oder OA substituierten Phenylrest,
Hal F, Cl, Br oder I
bedeuten
sowie deren Salze.

2. a) 6-[4-(4-(Benzimidazol-1-yl)-butyl)-piperazino]-1,4-benzodioxan;
b) 6-[4-(4-(5-Methoxy-indol-3-yl)-butyl)-piperazino]-1,4-benzodioxan;
c) 6-[4-(4-(5-Carbamoyl-indol-3-yl)-butyl)-piperazino]-1,4-benzodioxan
sowie
die Säureadditionssalze der genannten Verbindungen.

3. Verfahren zur Herstellung von 1,4-Benzodioxanderivaten der Formel I nach Anspruch 1 sowie von deren Salzen, dadurch gekennzeichnet, daß man eine Verbindung der Formel II
B-Q-X¹ II
worin
X¹ X oder NH₂ und
X Cl, Br, I, OH oder eine reaktionsfähig funktionell abgewandelte OH-Gruppe bedeuten und
B und Q die angegebenen Bedeutungen haben,
mit einer Verbindung der Formel III 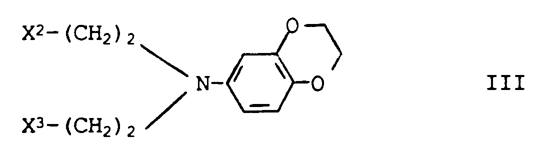 worin
X² und X³ gleich oder verschieden sein können und, falls X¹ = NH₂ ist, jeweils X, andernfalls zusammen NH bedeuten,
umsetzt
oder daß man eine Verbindung der Formel IV
B-Q-N(CH₂-CH₂-X)₂ IV
worin
X, Q und B die angegebenen Bedeutungen haben, mit einer Verbindung der Formel V 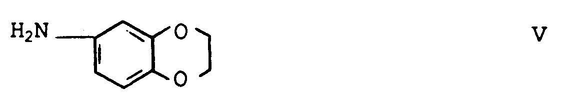 umsetzt
oder daß man eine sonst der Formel I entsprechende Verbindung, die jedoch anstelle der 1,4-Benzodioxangruppe eine 3,4-Dihydroxyphenylgruppe, wobei aber auch die beiden Hydroxygruppen zur Erhöhung der Reaktionsbereitschaft in entsprechend aktivierter Form vorliegen können, mit Ethandiol oder einem entsprechenden reaktiveren Derivat zu einer Verbindung der Formel I umsetzt
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt,
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt,
und/oder daß man gegebenenfalls eine OA-Gruppe unter Bildung einer OH-Gruppe spaltet und/oder eine Gruppe B in eine andere Gruppe B und/oder daß man eine erhaltene Base oder Säure der Formel I durch Behandeln mit einer Säure oder Base in eines ihrer Salze umwandelt.

4. Verfahren zur Herstellung pharmazeutischer Zubereitungen, dadurch gekennzeichnet, daß man eine Verbindung der Formel I nach Patentanspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der allgemeinen Formel I nach Patentanspruch 1 und/oder einem ihrer physiologisch unbedenklichen Salze.

6. Verwendung von Verbindungen der Formel I nach Patentanspruch 1 oder von deren physiologisch unbedenklichen Salzen zur Herstellung eines Arzneimittels.

7. Verwendung von Verbindungen der Formel I nach Patentanspruch 1 oder von deren physiologisch unbedenklichen Salzen bei der Bekämpfung von Krankheiten.
